(19) Europäisches Patentamt
European Patent Office
Office européen des brevets

(11) Publication number: **0 307 934 B1**

# (12) EUROPEAN PATENT SPECIFICATION

(45) Date of publication of patent specification: **24.11.93** (51) Int. Cl.$^5$: **C07C 25/22**, C07C 17/12

(21) Application number: **88115196.3**

(22) Date of filing: **16.09.88**

## (54) Process for the preparation of tricyclo 8.2.2.2. hexadeca 4,6,10,12,13,15 hexaene chlorinated in the benzenic rings.

(30) Priority: **18.09.87 IT 2195587**

(43) Date of publication of application:
**22.03.89 Bulletin 89/12**

(45) Publication of the grant of the patent:
**24.11.93 Bulletin 93/47**

(84) Designated Contracting States:
**AT BE CH DE ES FR GB LI NL SE**

(56) References cited:
**EP-A- 0 220 744**
**FR-A- 1 306 038**
**US-A- 3 288 728**
**US-A- 3 342 754**
**US-A- 4 166 075**

(73) Proprietor: **SYREMONT S.p.A.**
**31 Foro Buonaparte**
**I-20121 Milan(IT)**

(72) Inventor: **Ungarelli, Raffaele**
**1, via Tanaro**
**I-28069 Trecate Novara(IT)**
Inventor: **Beretta, Maurizio Augusto**
**75, via Valvassori Peroni**
**I-20133 Milan(IT)**
Inventor: **Sogli, Loris**
**2, via Ragazzi del 99**
**I- Novara(IT)**

(74) Representative: **Zumstein, Fritz, Dr. et al**
**Dr. F. Zumstein**
**Dipl.-Ing. F. Klingseisen**
**Bräuhausstrasse 4**
**D-80331 München (DE)**

**EP 0 307 934 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a process aimed to the preparation of tricyclo 8.2.2.2-hexadeca-4,6,10,12,13,15 hexaene (or (2,2)-paracyclophane) chlorinated in the aromatic benzenic rings.

More particularly, the present invention relates to a process for the preparation of tricyclo 8.2.2.2-hexadeca-4,6,10,12,13,15 hexaene chlorinated in the benzenic rings, with improved selectivity characteristics, by catalytic chlorination of the starting substrate (2,2)-paracyclophane) having the formula (I)

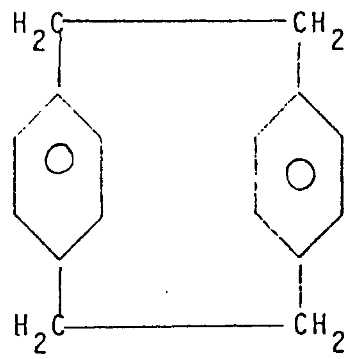

(I)

The chlorinated derivatives obtained by this process, having formula II:

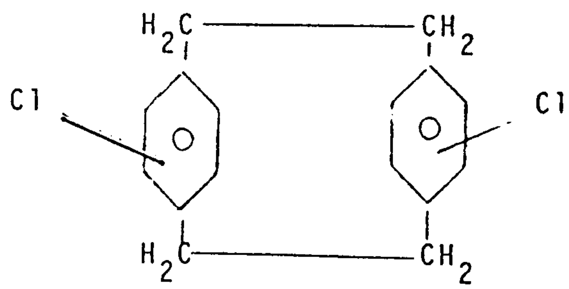

(II)

are products well known in the literature and are generally utilized as intermediates in the preparation of the corresponding poly-paraxylylene. Said polymers, and in particular poly-paraxylylene and its chlorinated derivatives, are advantageously utilized, for instance in the form of coating films in the field of the "conformal coating", obtained by application according to the vacuum vapor deposition technique, in the electronic field, etc.

As is known, the above mentioned technique of the vacuum vapor deposition requires the availability of a product having a high purity degree.

Various processes have been proposed for preparing chlorinated (2,2)-paracyclophane (II) and its derivatives.However, such known processes are not fully satisfactory and are not suitable for being adopted on an industrial scale, mainly due to the low selectivity of the process.

It is known from the literature (see for instance H. Wiegandt and P. Lantos - Industrial and Engineering Chemistry - Vol.43,9 pages 2167 to 2172 - September 1951; R. Mac-Mullin - Chemical Engineering Progress - Vol. 44,3 pages 183 to 188 - march 1948) that at the most a dihalogenated derivative selectivity equal to about 85% is obtained by catalytic chlorination of the benzene.

FR-A-1 306 038 discloses the preparation of halogenated di-para-xylylenes by halogenation of di-para-xylylene in the presence of a catalyst of the Friedel-Crafts type.

It is also known (see USA Patent No.4,166,075) that the selectivity in dichloro derivatives ( for example dichloroparaxylene) may be considerably improved, up to 95 - 96%, by the simultaneous use of metal halide as catalyst and of aliphatic hydrocarbon containing oxygenated functions, as co-catalyst.

The Applicant has found that, in the case of the clorination of the (2,2)-paracyclophane by using only the above-mentioned metal chloride catalyst, it is possible to obtain a selectivity in tetrachloroparacyclophane higher than 97 - 98%. It has been also proved the impossibility of obtaining dichloroparacyclophane with a selectivity higher than 90%, by using only the metal halide catalyst.

Now it has been found that the joined use of the chlorination catalyst and of the oxygenated hydrocarbon co-catalyst hereinafter described, allows to obtain dichloroparacyclophane with a selectivity ≧ 96%. Furthermore, in the mixture of the obtained dichloro-derivatives, those corresponding to a mono-chlorination of the benzenic ring, are sharply prevailing, contrary to the disclosure of US-A-4,166,075 where a high selectivity in dichloroparaxylene (95 - 96%) and a low selectivity (1 - 7%) in monochloroparaxylene are set forth.

It should be pointed out, moreover, that,owing to the aromatic mono-cyclic structure of the paraxylene substrate, there are in such a reference no teaching and/or suggestion useful for solving the problem of the symmetric chlorination (mono-chlorination) of the two benzenic aromatic rings present in 2,2-paracyclophane, which is the starting material of the present invention.

In other words, the above said technique does not disclose and does not give any suggestion as to the way to be followed for obtaining the mono-chlorinated derivative of the para-xylylene, which constitutes somehow a part of the (2,2)-paracyclophane molecule.

Object of the present invention is, therefore, to provide a process for preparing chlorinated (2,2)-paracyclophane having the above reported formula II, which process is characterized in that (2,2)-paracyclophane is reacted with gaseous chlorine by working in a halogenated organic suspending medium and/or a halogenated organic solvent, and in the presence of at least one chlorination catalyst, selected from the chlorides of Fe, Sb, Al, I, Cu and Sn, and in the presence of at least one co-catalyst having the formula

R - OH

wherein the symbol R represents a hydrocarbon or alkylcarboxylic radical, with linear or branched chain, containing from 1 to 6 carbon atoms the temperature being from -70 °C to 100 °C. Efficacious chloride catalysts proved to be: the chlorides of iron, aluminium, antimony, tin, iodine or mixtures thereof.

Efficacious co-catalysts proved to be: methanol, ethanol, (iso)propanol, butanol, formic acid, acetic acid, propionic acid, butyric acid or mixtures thereof.

The chlorination reaction is carried out, under stirring, at temperatures comprised between -70° and +100 °C, preferably comprised between 0° and +30 °C. The reaction is carried out in a solution and/or dispersion of (2,2)-paracyclophane in an inert halogenated, linear or branched aliphatic or aromatic hydrocarbon solvent, preferably $CCl_4$ or $CH_2Cl_2$, at atmosphere pressure for periods of time varying from 1 to 3 hours.

The catalyst is preferably produced in situ by the action in suspension of the chlorine on the metal. The amount used is such that:

$$\frac{\text{chlorination catalyst}}{\text{(2,2)-paracyclophane}} = \text{about } 0.001 - 0.01 \text{ in moles.}$$

The co-catalyst R - OH is added in amounts such that

$$\frac{\text{R - OH}}{\text{chlorination catalyst}} = \text{about } 1 - 30 \text{ in moles.}$$

In such a way it is possible to obtain high selectivity values for the single derivatives chlorinated in the benzenic rings having formula II. Particularly, for example, selectivity values in dichloro (2,2)-paracyclophane (G.C.titre) on the whole higher than 96% may be obtained and with a content of the derivative with a mono-chlorination in each one of its benzenic rings equal to or higher than 90%. In other words, the process of the present invention proved to be selectively suitable for the mono-chlorination of the benzenic rings present in (2,2)-paracyclophane (I). This is in contrast with what may be deduced from the above discussed known technique, the suggestions of which should dissuade one skilled in the art to expect the above results, especially in the case of the selective monochlorination of (2,2)-paracyclophane.

The present invention may be therefore considered as a surprising overcoming of a prejudice existing in the known art.

Furthermore the titre of the obtained dichloro-(2,2)-paracyclophane (> 96% G.C.) allows the remarkable advantage of the possibility to use the chlorinated raw product as such as obtained in the application processes, without the necessity of incurring the burdensome purification operations etc.

The starting product (2,2)-paracyclophane (8.2.2.2-hexadeca-4,6,10,12,13,15 hexaene) is a well known product available on the market or which may be prepared according to known processes.

Thus, for instance, (2,2)-paracyclophane may be obtained ("Organic Syntheses - Collective Volume 5 - John Wiley and Sons Inc. New York - London - Sydney - Toranto, 1973, pages 883 - 886) by the Hofmann elimination starting from p.methyl-benzyl-trimethyl-ammonium hydroxide obtained by reacting the corresponding bromide with silver oxide. The elimination is carried out in the presence of a basic compound and of an inert organic solvent (toluene).

When the reaction is finished, the mixture is treated for separating the chlorinated product, by operating according to conventional methods; by removing the catalyst, distillation of the solvent, washing etc.

The present invention is still further elucidated by the following examples, which however are to be constructed as merely illustrative. The term G.C. means gas-chromatography.

Example 1 has been carried out without the use of a co-catalyst in order to show the selectivity improvement of the yields obtained by the use of the co-catalyst in the case of the dichloroparacyclophane.

Examples 10 and 11 set forth the no influence of the co-catalyst presence, in the case of tetrachloroparacyclophane.

EXAMPLE 1 (Preparation of dichloroparacyclophane) (comparison)

36.5 g (0.175 moles) of (2,2)-paracyclophane, 0.05 g (0.0009 g-atoms) of Fe in powder and 0.5 l of $CCl_4$, were charged into a reactor having the capacity of 1 liter. Gaseous $Cl_2$ was then fed by maintaining the temperature at 18 - 20 °C, under stirring and at atmospheric pressure.

After a period of time of 1 hour, 25.6 g of chlorine were bubbled.

The solution was washed with alcaline $H_2O$ (5% of $NaHCO_3$), $CCl_4$ was evaporated at reduced pressure by obtaining 48.8 g of an amorphous white solid product having the titre G.C. 89. 2% of dichloroparacyclophane, corresponding to a yield of 89.8%.

The above data are listed in Table 1

EXAMPLES 2 - 9 (Preparation of dichloroparacyclophane in the presence of co-catalyst)

It was operated according to example 1 up to the feeding of 70% of the desired $Cl_2$,then the co-catalyst, was added and the addition of gaseous $Cl_2$ was completed.

The used reactants, the amounts thereof and the obtained products are listed in Table 1.

EXAMPLES 10 AND 11 ( Preparation of tetrachloroparacyclophane in the presence (11) and in the absence of the co-catalyst) (comparisons).

It was operated according to example 1 up to the feeding of 70% of the determined $Cl_2$, the co-catalyst was added and the chlorination was completed and the product recovered.

The obtained quantitative data are listed in Table 1.

TABLE 1 - Chlorination of (2,2)-paracyclophane

| Example No. | PCF g. | Solvent Type | Solvent l. | Catalyst Type | Catalyst g | Co-Catalyst Type | Co-Catalyst g | $Cl_2$ g. | Temperature °C | Time h | Obtained product Type | Obtained product g. | Obtained product lit % GC | Yield % |
|---|---|---|---|---|---|---|---|---|---|---|---|---|---|---|
| 1 | 36.5 | $CCl_4$ | 0.5 | Fe powder | 0.05 | -- | -- | 25.6 | 20 | 1 | Dichloro PCF | 48.8 | 89.2 | 89.8 |
| 2 | " | " | " | " | " | Acet.Ac | 0.5 | 25.5 | 18 | 1.25 | " | 48.5 | 96.5 | 96.6 |
| 3 | " | $CH_2Cl_2$ | " | " | " | Methanol | 0.55 | " | " | " | " | 47.9 | 96.1 | 94.9 |
| 4 | " | " | " | Sb | 0.11 | Ethanol | 0.8 | " | " | 1 | " | 47.3 | 96 | 93.7 |
| 5 | " | " | " | Al | 0.03 | Isoprop | 1.5 | " | 20 | 1.5 | " | 46.4 | 96.3 | 92.2 |
| 6 | " | $CCl_4$ | " | $I_2$ | 0.11 | Methanol | 0.55 | " | 18 | " | " | 46.2 | 96.4 | 91.9 |
| 7 | 20.8 | " | " | Sn | " | " | " | 14.5 | " | 3 | " | 25.9 | 95.9 | 89.7 |
| 8 | " | " | " | Cu | 0.03 | Ethanol | 0.5 | " | 20 | 2 | " | 26.1 | 96 | 90.4 |
| 9 | 36.5 | " | " | Fe Wool | 0.05 | Acet.Ac | 0.5 | 25.5 | 18 | 1.25 | " | 48.7 | 96.7 | 97.1 |
| 10 | " | " | " | Fe powder | 0.05 | --- | --- | 50.5 | 20 | 1.5 | Tetrachloro PCF | 60.9 | 99.1 | 99.4 |
| 11 | " | " | " | Fe Wool | " | " | " | 50.5 | " | 1.25 | " | 60.8 | 99 | 99.4 |

PCF = Paracyclophane

## Claims

**1.** A process for preparing a chlorinated (2,2)-paracyclophane having formula (II):

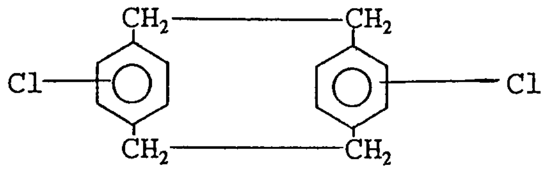

(II)

wherein (2,2)-paracyclophane (PCP) is reacted with gaseous chlorine in a halogenated organic suspending or solvent medium and in the presence of at least one chlorination catalyst, selected from the chlorides of Fe, Sb, Al, I, Cu and Sn, and in the presence of at least one co-catalyst having formula R-OH, where R represents a hydrocarbyl or alkyl-carboxylic radical, having a linear or a branched chain, containing from 1 to 6 carbon atoms, the temperature being from -70 ° C to 100 ° C.

**2.** The process according to claims 1 , characterized in that the reaction is carried out by operating in a chlorinated organic solvent and/or suspending medium at atmospheric pressure.

**3.** The process according to claim 2, characterized in that the reaction is carried out by operating in a solvent and/or suspending medium selected from $CCl_4$ and $CH_2Cl_2$, at a temperature comprised between 0 ° C and +30 ° C and at about the atmospheric pressure.

**4.** The process according to the preceding claims, characterized in that the chlorination catalyst is prepared in situ by the action of the gaseous chlorine on the metal in the reaction medium suspension.

**5.** The process according to the preceding claims, characterized in that the molecular ratio of the chlorination catalyst with respect to the starting (2,2)-paracyclophane is comprised between about 0.001 and 0.01 and the molar ratio of the co-catalyst with respect to the chlorination catalyst is comprised between about 1 and 30.

**6.** The process according to the preceding claims, characterized in that the chlorination catalyst is ferric chloride.

**7.** The process according to the preceding claims, characterized in that the co-catalyst is selected from the group consisting of methyl alcohol, ethyl alcohol, isopropyl alcohol, acetic acid or mixture thereof.

## Patentansprüche

**1.** Verfahren zur Herstellung eines chlorierten (2,2)-para-Cyclophans der Formel (II)

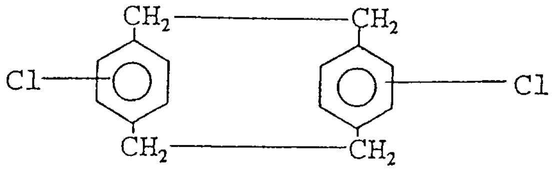

(II)

worin (2,2)-para-Cyclophan (PCP) mit gasförmigem Chlor in einem halogenierten organischen Suspendier- oder Lösungsmedium in Gegenwart zumindest eines Chlorierungskatalysators, ausgewählt unter den Chloriden von Fe, Sb, Al, I, Cu und Sn, sowie in Gegenwart zumindest eines Co-Katalysators der Formel R-OH, worin R einen Hydrocarbyl- oder Alkylcarboxylrest mit einer linearen oder verzweigten Kette von 1 bis 6 Kohlenstoffatomen bedeutet, bei einer Temperatur im Bereich von -70 bis 100 ° C umgesetzt wird.

2. Verfahren gemäß Anspruch 1, dadurch gekennzeichnet, daß die Umsetzung durchgeführt wird, indem man in einem chlorierten organischen Lösungsmittel und/oder Suspendiermedium bei Atmosphärendruck arbeitet.

3. Verfahren gemäß Anspruch 2, dadurch gekennzeichnet, daß die Umsetzung durchgeführt wird, indem man in einem Lösungsmittel und/oder Suspendiermedium, ausgewählt unter $CCl_4$ und $CH_2Cl_2$, bei einer Temperatur zwischen 0 und +30°C und bei etwa Atmosphärendruck arbeitet.

4. Verfahren gemäß den vorhergehenden Ansprüchen, dadurch gekennzeichnet, daß der Chlorierungskatalysator in situ durch Einwirken von gasförmigem Chlor auf Metall in der Reaktionsmediumsuspension hergestellt wird.

5. Verfahren gemäß den vorhergehenden Ansprüchen, dadurch gekennzeichnet, daß das Molverhältnis von Chlorierungskatalysator zu dem Ausgangs-(2,2)-para-Cyclophan zwischen etwa 0,001 und 0,01 beträgt, und das Molverhältnis des Co-Katalysators zu dem Chlorierungskatalysator zwischen etwa 1 und 30 liegt.

6. Verfahren gemäß den vorhergehenden Ansprüchen, dadurch gekennzeichnet, daß der Chlorierungskatalysator Ferrichlorid ist.

7. Verfahren gemaß den vorhergehenden Ansprüchen, dadurch gekennzeichnet, daß der Co-Katalysator unter Methylalkohol, Ethylalkohol, Isopropylalkohol, Essigsäure oder Mischungen hiervon ausgewählt wird.

## Revendications

1. Procédé de préparation de (2,2)-paracyclophane chloré représenté par la formule (II):

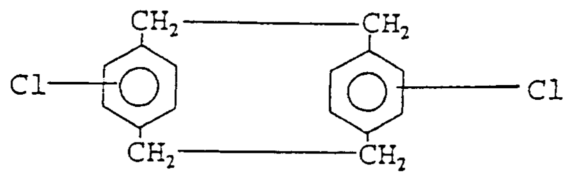

(II)

dans laquelle du (2,2)-paracyclophane (PCP) est traité avec du chlore gazeux dans un milieu solvant ou de suspension organique halogéné et en présence d'au moins un catalyseur de chloration, choisi parmi les chlorures de Fe, Sb, Al, I, Cu et Sn et en présence d'au moins un co-catalyseur représenté par la formule R-OH, dans laquelle R est un radical hydrocarboné ou alkylcarboxylique, à chaîne droite ou ramifiée, contenant 1 à 6 atomes de carbone, à une température comprise entre -70°C et +100°C.

2. Procédé selon la revendication 1, caractérisé en ce que la réaction est réalisée à la pression atmosphérique dans un milieu solvant et/ou de suspension organique chloré.

3. Procédé selon la revendication 2, caractérisé en ce que la réaction est réalisée dans un milieu solvant et/ou de suspension choisi parmi $CCl_4$ et $CH_2Cl_2$, à une température comprise entre 0°C et +30°C, au voisinage de la pression atmosphérique.

4. Procédé selon l'une quelconque des revendications précédentes, caractérisé en ce que le catalyseur de chloration est préparé in situ par l'action du chlore gazeux sur le métal dans la suspension du milieu réactionnel.

5. Procédé selon l'une quelconque des revendications précédentes, caractérisé en ce que le rapport moléculaire du catalyseur de chloration/(2,2)-paracyclophane de départ est compris entre environ 0,001 et 0,01 et le rapport molaire du co-catalyseur/catalyseur de chloration est compris entre environ 1 et 30.

6. Procédé selon l'une quelconque des revendications précédentes, caractérisé en ce que le catalyseur de chloration est du chlorure ferrique.

7. Procédé selon l'une quelconque des revendications précédentes, caractérisé en ce que le co-catalyseur est choisi dans le groupe consistant en méthanol, éthanol, isopropanol, acide acétique ou un mélange de ceux-ci.